# EUROPEAN PATENT APPLICATION

(11) **EP 0 704 529 A2**
(43) Date of publication of application: **03.04.1996**
(21) Application number: 95500099.7
(22) Date of filing: 06.07.1995
(51) Int. Cl.: C12N 15/41, A61K 39/125, C07K 14/085, C12N 15/86

(54) **Vaccine against rabbit hemorrhagic disease virus**

(30) Priority: 06.07.1994 ES 9401512
(71) Applicant: UNIVERSIDAD DE OVIEDO, E-33003 Oviedo (ES)
(72) Inventor: Francisco Parra c/o Universidad de Oviedo, Avda.de Julian Claveria s/n 33006 Oviedo (ES); Marin, Maria S., E-09268 Villagalijo (ES); Martin, José M., E-33005 Oviedo (ES); Casais Rosa Universidad de Oviedo Facultad de Med., Claveria,s/n 33006 Oviedo (ES); Boga, José A., E-33212 Gijon (ES)
(74) Representative: Ibanez, José Francisco

(57) **Abstract**

Production of the structural antigen (VP60) of the virus of the hemorrhagic disease of the rabbits, RHDV, and its use as a vaccine against said disease. The production of the antigen and the method of vaccination comprise the following stages: a) isolation of the gene that code the structural protein (VP60) of the RHDV; b) cloning of said gene in the baculovirus transfer vector pAcYM1; c) obtention and isolation of the recombinant baculovirus AcVP60; d) infection of Sf9 insect cells with the recombinant baculovirus AcVP60; e) obtention and isolation of the protein VP60 produced by the recombinant baculovirus AcVP60; f) inoculation of the rabbits with the antigen.

## Description

The invention relates to the production of the structural antigen of the virus of the hemorrhagic disease of the rabbits, and its use as a vaccine against said disease.

The hemorrhagic viral disease of the rabbits produces big economic losses in the rabbit farming industry and has decreased the population of rabbits in fields and forests with serious damage for the hunting groups.

Since the appearance of the disease in 1984 in the Chinese province of Jiangsu (S.J. Liu, H.P. Xue, B.Q. Pu and N.H. Quian. 1984. Anim. Husb. Vet. Med. 16:253-255), it has been extended for all Asia, Europe and North Africa.

The etiologic agent, known as rabbit hemorrhagic disease virus (RHDV), has been included recently in the *Caliciviridae* family. The members of this family present the following common characteristics:
- Virions of 25-40 nm, with icosahedral symmetry and without lipid envelope.
- A capsid formed by units of a polypeptide which mass ranges between 60 and 71 kdalton.
- A genome of single stranded RNA of positive polarity (7.5 kb), joined to a protein (Vpg) in its 5' end and polyadenylated in its 3' end.

Also, the RHDV presents a subgenomic RNA of 2.4 kb, encapsidated and coterminal with the 3' end of the genome, with capacity for coding the structural protein.

At the present time, several commercial vaccines exist, known as tissue vaccines, based on the use of extracts from organs of experimentally infected rabbits, in which the virulent agent is thermally or chemically inactivated. The efficiency of these preparations has been tested in laboratory (J.E. Peeters, D. Vandergheynst and R. Geeroms. 1992. Cuni-Sciences 7:101-106) and in the field, but they present serious inconvenients of antigenic variability and biological risk due to their mode of elaboration.

The invention relates to the production of the structural antigen (VP60) of the rabbit hemorrhagic disease virus, RHDV (Isolate AST/89), and its use as a vaccine against said disease.

The obtention of the protein VP60 in an heterologous system of insect cells would provide big quantities of a product with defined antigenic characteristics, avoiding the variability and the biological risk derived of the use of the virulent agent, as it happens in the elaboration of the tissue vaccines. At the same time, the recombinant product would allow a better control of quality and standardization of the vaccine preparation.

A first objective of this invention is to provide a method for the preparation of a recombinant vaccine for the immunization of rabbits against the hemorrhagic disease caused by the RHDV, in which the vaccine antigen VP60 is produced by means of DNA recombination techniques, that comprises the following stages:
a) Construction of a complementary DNA (cDNA) of the gene of the protein VP60;
b) Use of a transfer vector for introducing the VP60 gene of the RHDV in the genome of a baculovirus by means of an homologous recombination process;
c) Use of the recombinant baculovirus for infecting cultures of the Sf9 insect cell line or other cell lines, or larvae of insects that were permissive hosts for the replication of the virus of the nuclear polyhedrosis of *Autographa californica*, during the time and required conditions for the production of the protein VP60 of the RHDV, and
d) Obtention of the protein VP60, free of other proteins of the RHDV, starting from extracts of cell cultures or larvae of insects infected by the recombinant baculovirus.

Another objective of the invention is to provide a composition that comprises the baculovirus AcVP60 (CNCM deposit number I-1441), usable as a vaccine against the hemorrhagic viral disease of the rabbits.

A form of execution of the invention comprises the following stages:

### A) Cloning of the gene of the VP60 of the RHDV.

For cloning the gene of the VP60 of the RHDV, viral particles are isolated from livers infected by the virus and from these particles obtained their genomic and subgenomic RNAs are obtained. These RNAs are separated by zonal centrifugation in sucrose gradients and the subgenomic RNA is used as template for the synthesis of a complementary DNA (cDNA). Then *Bgl*II adapters are added. The resulting cDNA fragment comprises the nucleotides 10 to 1834 of the subgenomic RNA, to which the nucleotidic sequences GATCTTCCAT and CAGATC have been added to the 5' end and to the 3' end respectively.

### B) Construction of the transfer vector of the baculovirus containing the gene of the VP60.

For the construction of the transfer vector of the baculovirus that contains the gene of the VP60, the cDNA obtained in the previous stage is cloned in the *Bam*HI site of the pAcYM1 baculovirus transfer vector (Y. Matsuura, R.D. Posee, H.A. Overton and D.H.L. Bishop. 1987. J. Gen. Virol. 68:1233-1250). The orientation of the gene inserted in the resulting vector pAcVP60 is tested by restriction and sequencing analysis.

### C) Obtention of the recombinant baculovirus containing the gene of the VP60: isolation and characterization.

The vector pAcVP60 obtained in the previous stage, is cotransfected with the linearized DNA of the baculovirus AcMNPV.*Lac*Z, in the Sf9 insect cells. The recombinant baculovirus present in the culture medium after more than 20 hours of incubation are separated from the parentals by plate assays of their white (recombinant) or blue (parentals) phenotype in presence of X-Gal (5-bromine-4-chlorine-3-indolyl-β-D-galactopiranoside).

Their capacity to produce VP60 is also investigated by indirect immunofluorescence using a hyperimmune serum against the protein VP60. In this way the recombinant baculovirus AcVP60 is obtained (CCNCM deposit number I-1441) which is used in the next stages for producing the recombinant protein VP60 in the Sf9 insect cells.

### D) Obtention of the recombinant protein VP60.

For the obtention of big quantities of recombinant protein VP60, the cells Sf9 are infected with recombinant baculovirus AcVP60 at a 1:5 multiplicity of infection maintaining the culture for 3 days at 28° C. Then the cells are harvested, washed with saline-phosphate buffer and broken by freezing and thawing. The cell extracts are clarified by low speed centrifugation and the quantity of total protein in the extract free of cells is determined by colorimetry. In order to confirm the production of the recombinant protein the extracts are analyzed in a polyacrylamide gel at 10% in presence of SDS and the quantity corresponding to the protein VP60 is evaluated by densitometry means.

The efficiency of the recombinant protein VP60 as a vaccine against the hemorrhagic viral disease of the rabbits is evaluated by means of intradermic inoculation of rabbits with increasing doses of antigen, from 1 µg to 250 µg/animal. Blood samples of all the animals are taken before the immunization, as well as 22 days after. Lapsed 23 days from the immunization, the vaccinated animals and an equivalent number of controls without being immunized are exposed to the RHDV of the disease.

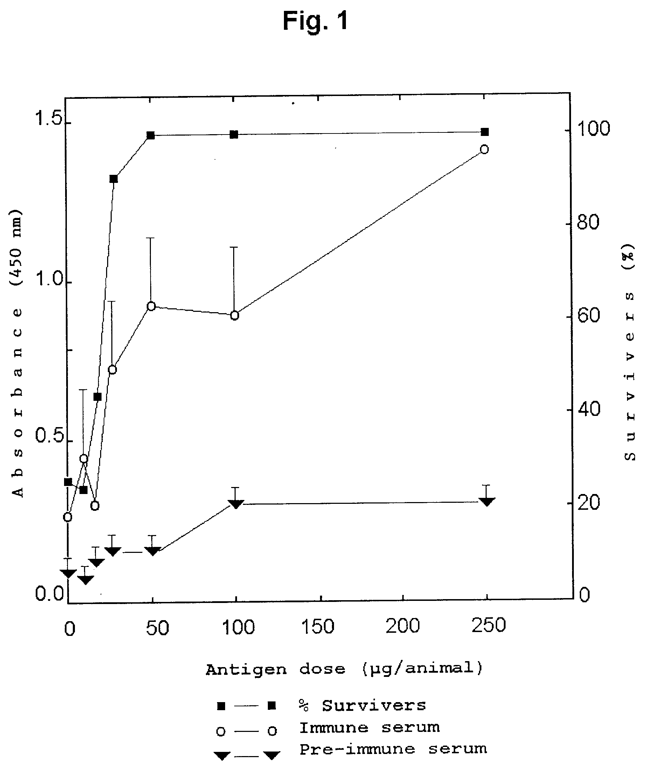

The obtained results are shown in the diagram of the attached Figure 1, where the percentage of surviving animals in relation to the dose of antigen used for the immunization is represented, as well as the evolution of the immune response of the animals. An increment of the survival of the animals with the used dose of antigen is observed, in parallel with an increment in the titre of anti-VP60 antibodies. For the detection of antibodies against the recombinant protein in the serum of the animals an indirect ELISA, is employed using partially purified RHDV as antigen.

For the elaboration of the vaccine preparation, the cell extract obtained in previous stage D is diluted with saline-phosphate buffer until obtaining a concentration of VP60 of 50 µg/ml. This solution, without other immunity adjuvants, is used for the immunization of the rabbits by means of a single minimum intradermic dose of 1 ml. The elected quantity of antigen was selected considering that the half immunizing dose ranges between 10 and 25 µg/animal.

The following example refers, without restrictive scope, to a preferable way of practical execution of the invention, describing the cloning of the gene that codes the protein VP60 of the RHDV, its expression in an heterologous system of insect cells and the use of the recombinant antigen for the vaccination of rabbits against the hemorrhagic disease.

### EXAMPLE

### A) cloning of the gene of the VP60 antigen of the RHDV.

Virions of the RHDV were initially purified by means of several stages of ultracentrifugation, using as starting material extracts from the liver of experimentally infected rabbits. The genetic material of the virions was extracted using the guanidine isothiocyanate method. The subgenomic RNA messenger (RNAm) was separated from the viral genome by zonal centrifugation in sucrose gradients, using it then as template for synthesizing a complementary DNA (cDNA) in presence of an oligo dT primer (J.A. Boga et al. 1992. Virus Res. 26:33-40). The aminoacidic sequence of the amino terminal end of the protein VP60 allowed to locate the coding region of this antigen between the nucleotides 10 and 1834 of the subgenomic RNAm of the AST/89 isolated of the RHDV (EMBL data base, access number Z24757).

### B) Construction of the transfer vector pAcVP60.

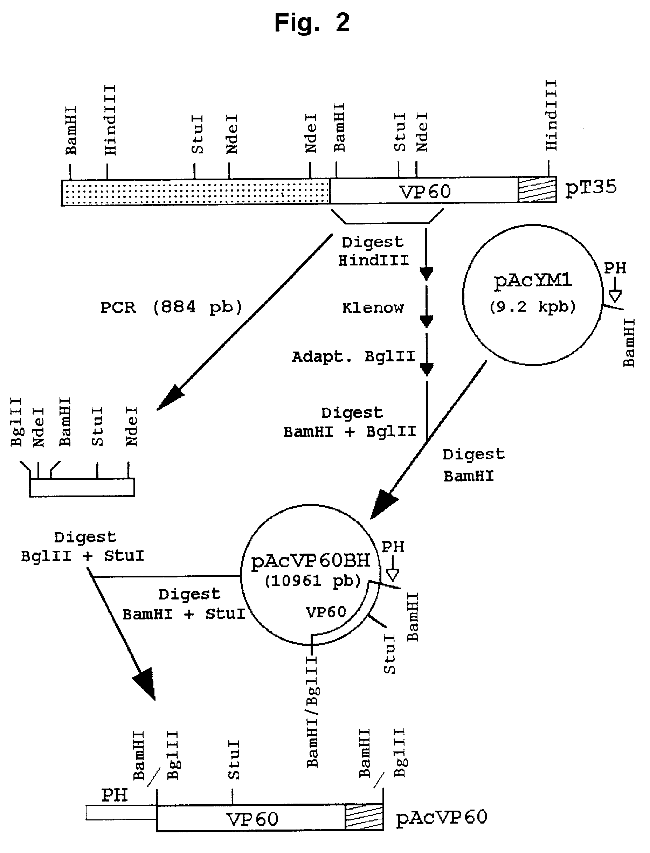

The adopted procedure is represented in the diagram of the attached Figure 2. A *Hind*III fragment (4147 pb) from the clone of cDNA pT35, to which *Bgl*II adapters had been added in the ends, was digested with *Bam*HI and *Bgl*II enzymes. The resulting fragment of 1731 pb was inserted in the *Bam*HI site of the vector pAcYM1. The correct orientation of the insert present in the recombinant plasmid pAcVP60BH was investigated by restriction analysis. From an 884 pb cDNA fragment, obtained from the cDNA T35 by polymerase chain reaction (PCR), a restriction fragment *Bgl*II/*Stu*I was obtained which was inserted in the vector pAcVP60BH digested with the *Bam*HI and *Stu*I enzymes. The resulting transfer vector, known as pAcVP60, was tested by restriction and sequencing analysis.

### C) Obtention of the recombinant baculovirus AcVP60.

In order to carry out the transfection 24 µl of a mixture containing 200 ng of DNA of the baculovirus AcMNPV.*Lac*Z linearized with the *Bsu*36I enzyme, 2 µg of the plasmid pAcVP60 and 8 µl of lipofectin are added to a suspension of 10⁶ Sf9 cells in 1 ml of TC-100 medium. The resulting mixture is incubated for 7 hours at 28° C and is added 1 ml of culture medium that contains fetal calf serum at 5%. 20 hours later the culture medium is changed for fresh medium and after 72 hours the medium is harvested in order to separate the recombinant baculovirus AcVP60 from the parentals by plate assays in presence of X-Gal. The expression of the VP60 antigen in the cells infected by the recombinant baculovirus is investigated by indirect immunofluorescence using a hyperimmune serum against the protein VP60.

### D) Production of the recombinant VP60 protein.

For the obtention of sufficient amounts of the recombinant VP60 protein, single layers of 2 x 10⁷ Sf9 cells are infected with 10⁸ recombinant AcVP60 baculovirus and the culture in TC-100 medium containing 5% of fetal calf serum is kept for 3 days at 28° C. Then the cells are harvested, washed with saline-phosphate buffer and broken after three successive processes of freezing and thawing. The cell extracts are clarified by centrifugation at 1500 x g for 10 minutes and the total protein of the extract is determined by colorimetry. In order to quantify the proportion of recombinant VP60 protein in relation to the total protein of the cell extracts, these are analyzed by electrophoresis in polyacrylamide gels with SDS, the amount corresponding to the protein VP60 being determined by densitometry means.

The cell extract obtained in the previous stage is diluted with saline-phosphate buffer until reaching a concentration of VP60 of 50 µg/ml.

In order to immunize the rabbits a single dose of 1 ml of this preparation is directly applied intradermally without immunity adjuvants.

## Claims

1. Method for the preparation of a recombinant vaccine for the immunization of rabbits against the hemorrhagic disease caused by the RHDV, in which the vaccine antigen VP60 is produced by DNA recombination techniques, characterized in that it comprises the following stages:
a) Construction of a complementary DNA (cDNA) of the gene of the protein VP60;
b) Use of a transfer vector for introducing the VP60 gene of the RHDV in the genome of a baculovirus by means of an homologous recombination process;
c) Use of the recombinant baculovirus for infecting cultures of the Sf9 insect cell line or other cell lines, or larvae of insects that were permissive hosts for the replication of the virus of the nuclear polyhedrosis of *Autographa californica*, during the time and required conditions for the production of the protein VP60 of the RHDV, and
d) Obtention of the protein VP60, free of other proteins of the RHDV, from extracts of cell cultures or larvae of insects infected by the recombinant baculovirus.

2. Method, according to claim 1, characterized in that it comprises the following stages:
a) Construction of a cDNA of the gene of the protein VP60 from the subgenomic RNAm of the isolated AST/89 of RHDV, to which the nucleotidic sequences GATCTTCCAT and CAGATC have been added to the 5' end and to the 3' end respectively.
b) Use of the transfer vector pAcYM1 for introducing the VP60 gene of the RHDV in the genome of the virus of the nuclear polyhedrosis of *Autographa californica* AcMNPV.*Lac*Z, generating the recombinant baculovirus AcVP60 (CNCM deposit number I-1441), by means of an homologous recombination process with the gene of the polyhedrin;
c) Use of the recombinant baculovirus AcVP60 generated according to the previous stage, in order to infect cultures of the Sf9 insect cell line or other cell lines, or larvae of insects that were permissive hosts for the replication of the virus of the nuclear polyhedrosis of *Autographa californica*, during the time and required conditions for the production of the protein VP60 of the RHDV, and
d) Obtention of the protein VP60, free of other proteins of the RHDV, starting from extracts of cell cultures or insects infected by the recombinant baculovirus AcVP60.

3. Method, according to claims 1 or 2, characterized in that the vaccine antigen VP60 is produced free of other proteins of RHDV during the replication of the recombinant baculovirus AcVP60 in cell lines in culture, or in larvae of insects that were permissive hosts for the selected baculovirus.

4. The baculovirus AcVP60 (CNCM deposit number I-1441).

5. A composition that comprises the baculovirus AcVP60 (CNCM deposit number I-1441).

6. A vaccine against the hemorrhagic viral disease of the rabbits that comprises the baculovirus AcVP60 (CNCM deposit number I-1441).

7. Use of the baculovirus AcVP60 (CNCM deposit number I-1441) in the preparation of a vaccine against the hemorrhagic viral disease of the rabbits.

8. Use of the protein VP60 in the preparation of a vaccine against the hemorrhagic viral disease of the rabbits.
